# EUROPÄISCHE PATENTSCHRIFT

(11) **EP 3 479 872 B1**
(45) Veröffentlichungstag und Bekanntmachung des Hinweises auf die Patenterteilung: **12.08.2020**
(21) Anmeldenummer: 18203733.3
(22) Anmeldetag: 31.10.2018
(51) Int. Cl.: A61N 2/00, A61N 2/02

(54) **VORRICHTUNG ZUR MAGNETFELDBEHANDLUNG VON PERSONEN AUF EINER BEHANDLUNGSLIEGE UND/ODER EINEM BEHANDLUNGSSTUHL**
DEVICE FOR MAGNETIC FIELD TREATMENT OF PEOPLE IN A TREATMENT BED AND/OR TREATMENT CHAIR
DISPOSITIF DE TRAITEMENT PAR CHAMP MAGNÉTIQUE DES PERSONNES SUR UN LIT DE TRAITEMENT ET / OU SUR UNE CHAISE DE TRAITEMENT

(30) Priorität: 03.11.2017 DE 102017125678
(43) Veröffentlichungstag der Anmeldung: 08.05.2019
(73) Patentinhaber: Prof. Dr. Fischer AG, 9497 Eschen (LI)
(72) Erfinder: FISCHER, Gerhard, 9435 Heerbrugg (CH)
(74) Vertreter: Riebling, Peter

(56) Entgegenhaltungen:
- WO-A2-2009/156117
- DE-A1- 3 137 455
- ES-A6- 2 023 590
- US-B1- 6 213 933

## Beschreibung

Die Erfindung betrifft eine Vorrichtung zur Magnetfeldbehandlung von Personen auf einer Behandlungsliege und/oder einem Behandlungsstuhl mit mindestens einer Magnetspule.

Der Einsatz hochintensiver dynamischer Magnetfelder zur Erzeugung von Aktionspotentialen im zentralen aber auch peripheren Nervensystem ist in der Medizin von wachsendem Interesse. So ist die repetitive transkranielle Magnetstimulation rTMS nicht nur ein anerkanntes Verfahren in der Behandlung therapieresistenter Major Depressions, sondern wirkt auch bei Neuropathien, Fibromyalgie, Migräne, L-Dopa-bedingten Dyskinesien bei Morbus Parkinson oder auch bei Bewegungsstörungen nach Erkrankungen wie MS und ALS. Mit der repetitiven peripheren Magnetstimulation rPMS hat sich inzwischen sich in Form einer transpelvinen Magnetstimulation rTPM eine wirksame Therapieoption in der Behandlung verschiedener Spektren einer Harninkontinenz entwickelt. Auch hinsichtlich einer Inkontinenz nach Prostatektomie, dem Pelvic-Pain-Syndrom, Post-Partum-Schäden, einer erektile Dysfunktion sowie der sexuellen Dysfunktion der Frau zeichnen sich positive Wirkungen ab.

Zur Durchführung einer rPMS / rTPM steht auf der Grundlage eigener RCT-Studien ein Medizintechniksystem (PelviCenter) zur Verfügung. Das Pelvicenter knüpft dabei an das grundsätzliche Wirkprinzip einer Magnetstimulation des Beckenbodens an, zu dem in der Datenbank Pubmed (Medline) inzwischen schon eine Vielzahl von klinischen Studien mit teilweise deutlichen oder signifikanten Therapieeffekten in der Behandlung einer Stress-, Drang- und Mischinkontinenz indexiert sind. Auffällig ist dabei, dass sich die jeweiligen Studiendesigns zur Reizkonfiguration weitgehend ähneln und Erkenntnisse aus der Trainingsphysiologie der Skelettmuskulatur nicht immer umgesetzt werden.

Da es sich bei der komplexen Schichtarchitektur des Beckenbodens um Skelettmuskeln handelt, erscheint es notwendig, Stimulationseffekte im Hinblick auf den Einsatz optimaler Reizparameter zu quantifizieren. Hierzu hat die Prof. Dr. Fischer AG eine Grundlagenstudie an der Universität der Bundeswehr In München-Neubiberg, Institut für Sportwissenschaften und Sport, durchführen lassen, denen bereits eine Untersuchung der magnetstimulatorischen Reizschwelle (Verschmelzungsfrequenz) eines günstigen Muskeltetanus an der Wadenmuskulatur unter Ultraschallkontrolle vorausgegangen war.

Ein Verfahren und eine Vorrichtung zur Magnetfeldbehandlung mit einer im Behandlungsstuhl verschiebbar angetriebenen Magnetspule sind beispielsweise mit dem Gegenstand der DE 10 2016 116 399 A1 bekannt geworden. Allerdings vermag diese Druckschrift nicht die Behandlung einzelner, entfernt voneinander befindliche Körperregionen einer Person zu verwirklichen, weil die verschiebbar angetriebene Magnetspule lediglich im unteren Lehnenbereich und im Sitzbereich des Behandlungsstuhles angeordnet ist. Die Beeinflussung unterschiedlicher Körperregionen während der Verschiebung der Magnetspule mit unterschiedlichen Magnetfeldern, deren Stärke und Frequenz sowie Verweildauer variieren kann, ist aus dieser Druckschrift nicht zu entnehmen.

Die DE 10 2011 014 291 A1 zeigt ein weiteres Verfahren und eine Vorrichtung, bei der zu entnehmen ist, dass unterschiedliche Körperorgane und Körperregionen mit unterschiedlich starken Magnetfeldern bei unterschiedlicher Frequenz und Verweildauer behandelt werden können.

DE ES 2 023 590 A6 offenbart eine Vorrichtung zur magnetischen Massage-Therapie, welche eine oder mehrere verschiebbare oder verschwenkbare Feldspulen aufweist. Die Feldspulen werden durch Impulse mit vorgegebener oder veränderlicher Frequenz angesteuert.

Ähnliche Vorrichtungen zur Magnetfeldbehandlung sind in der DE 31 37 455 A1, WO 2009/156117 A2 oder US 6 213 933 B1 beschrieben, wobei hier die Frequenz und teilweise die Intensität des Magnetfeldes steuerbar ist. Die Steuerung der Amplitude, der Frequenz oder der Verweildauer einer oder mehrerer Magnetspulen während des Verfahrweges auf der Liege oder dem Behandlungsstuhl ist aus diesen Druckschriften jedoch nicht zu entnehmen. Der Erfindung liegt die Aufgabe zugrunde, eine Vorrichtung zur Magnetfeldbehandlung von Personen auf einer Behandlungsliege und/oder einem Behandlungsstuhl so weiterzubilden, dass während der Behandlungsdauer unterschiedliche Körperregionen der Person mit unterschiedlichen Frequenzen und/oder Amplituden und/oder Behandlungszeiten und/oder Wiederholzeiten behandelt werden können.

Zur Lösung der gestellten Aufgabe ist die Erfindung durch die technische Lehre des Anspruches 1 gekennzeichnet.
Erfindungsgemäß wird Magnetfeldapplikator zur Magnetfeldbehandlung von Personen auf einer Behandlungsliege und/oder einem Behandlungsstuhl mit mindestens einer Magnetspule vorgeschlagen, wobei die mindestens eine Magnetspule mindestens teilweise über die Länge der Behandlungsliege und/oder des Behandlungsstuhls längsverschiebbar ausgebildet ist und ein den Körper der Person mindestens teilweise durchdringendes Magnetfeld erzeugt, wobei während der Längsverschiebung der mindestens einen Magnetspule die Stärke (=Amplitude) des mindestens einen Magnetfeldes und/oder dessen Frequenz und/oder dessen Impulsform und/oder dessen Behandlungsdauer und/oder dessen Behandlungswiederholfrequenz positionsabhängig in Abhängigkeit von der Region des Körpers der Person gesteuert wird. Die verschiebbare Magnetspule ist mit einem Positionssensor verbunden, der die aktuelle Stellung und Position der Magnetspule in Bezug zur behandelten Körperregion erfasst. Das so erfasste Sensorsignal wird einem Generator eingegeben, der die Amplitude des Magnetfeldes und/oder die Frequenz und/oder die Wiederholdauer und/oder die Behandlungszeit und/oder die Impulsform steuert.

Der Anwendungsbereich der Erfindung betrifft demnach alle Körperregionen einer auf der Behandlungsliege oder einer auf dem Behandlungsstuhl sitzenden Person, wobei im Vordergrund der Erfindung die Behandlung der Beckenregion steht, die in der folgenden Beschreibung auch als "Pelvis" bezeichnet ist.

Das Becken (Pelvis) ist der Körperabschnitt unterhalb des Bauchs und oberhalb der Beine. Man unterscheidet beim Menschen zwischen einem großen und einem kleinen Becken (Pelvis major und Pelvis minor). Das große Becken liegt zwischen beiden Darmbeinschaufeln oberhalb der Beckeneingangslinie (Linea terminalis) und gehört eigentlich zum Bauchraum.

Der Gegenstand des Anspruchs 1 betrifft demnach eine Vorrichtung, bei dem/der während der Längsverschiebung der mindestens einen Magnetspule die Stärke des mindestens einen Magnetfeldes und/oder dessen Frequenz positionsabhängig in Abhängigkeit von der Region des Körpers der Person gesteuert wird.

Eine bevorzugte Weiterbildung der Vorrichtung sieht vor, dass während der Längsverschiebung die Verweildauer an einer bestimmten Position der mindestens einen längsverschiebbaren Magnetspule positionsabhängig in Abhängigkeit von der Region des Körpers der Person gesteuert wird.

Eine weitere bevorzugte Weiterbildung der Vorrichtung sieht vor, dass während der Längsverschiebung die Wiederholfrequenz der Einwirkung des Magnetfeldes an einer bestimmten Position der mindestens einen längsverschiebbaren Magnetspule positionsabhängig in Abhängigkeit von der Region des Körpers der Person gesteuert wird

Es ist deshalb mindestens eine ortsveränderbare Magnetspule vorhanden, die mindestens teilweise über die Länge der Behandlungsliege und/oder des Behandlungsstuhls längsverschiebbar ausgebildet ist. Dies bedeutet in einer ersten Ausführungsform, dass die Magnetspule beispielsweise im Mittenbereich der Behandlungsliege und/oder des Behandlungsstuhls angeordnet ist und dort (nur) im Mittenbereich auch verschiebbar ist.

Nach einer anderen Ausführung ist vorgesehen, dass eine solche Magnetspule (nur) im Kopfbereich und/oder im Beinbereich der Behandlungsliege oder des Behandlungsstuhls angeordnet ist und nur auf diesem Bereich längsverschiebbar ist.

Dieser allgemeine Erfindungsgedanke sieht demnach die Verschiebbarkeit mindestens einer Magnetspule in bestimmten Bereichen einer Behandlungsliege und/oder eines Behandlungsstuhls vor, wobei deren physikalisches Verhalten (Frequenz, Amplitude, Impulsform, Behandlungsdauer, Wiederholfrequenz) in Abhängigkeit von der zu behandelnden Körperregion gesteuert wird.

In einer Weiterbildung ist vorgesehen, dass nur eine einzige Magnetspule vorhanden ist, die über die gesamte Länge der Behandlungsliege und/oder des Behandlungsstuhls längsverschiebbar ausgebildet ist.

Anstatt der Verwendung einer einzigen Magnetspule, die in einem bestimmten Bereich der Behandlungsliege oder des Behandlungsstuhls längsverschiebbar ist oder auch anstatt der weiteren Ausführungsform, dass mehrere Magnetspulen nach dem Gegenstand des Anspruches 1 verwendet werden, die jedoch auch nur in den ihnen zugeordneten Bereich längsverschiebbar sind, beansprucht eine bevorzugte weitere Ausführungsform, dass eine einzige Magnetspule vorhanden ist, die über die gesamte Länge der Behandlungsliege und/oder des Behandlungsstuhls längsverschiebbar ausgebildet ist.

Damit ist es erstmals möglich, eine Hochstrom-Magnetbehandlung mit einem einzigen Generator für mindestens eine Magnetfeldspule vorzusehen, deren Anwendungsbereich sich über die gesamte Länge der Behandlungsliege erstreckt.

Der Begriff "gesamte Länge" muss nicht identisch sein mit der Länge der Behandlungsliege oder der wirksamen Länge des Behandlungsstuhls, weil es nur darauf ankommt, möglichst alle Körperbereiche der zu behandelnden Person von dem Magnetfeld durchsetzen zu lassen. Daher kann die Verschiebungslänge der Magnetspule kürzer sein als die "gesamte Länge der Behandlungsliege", weil es ausreicht, die Verschiebungslänge der Magnetspule so zu wählen, dass sichergestellt ist, dass alle zu beaufschlagenden Körperteile der zu behandelnden Person auch von dem Magnetfeld der einzigen Magnetspule durchdrungen werden.

Die Flussdichte des Magnetfeldes nimmt sehr stark mit dem Abstand von der magnetfelderzeugenden Oberfläche der Spule ab.

Daher wird es in einer Weiterführung der Erfindung vorgeschlagen, dass die Oberkante der Magnetspule mit der Ebene der Liegefläche annähernd fluchtet, so dass stets dafür gesorgt ist, dass die zu behandelnde Person praktisch mit ihrer der Liegefläche zugewandten Körperoberfläche auf der Oberseite der Magnetspule aufliegt.

Es kann sogar vorgesehen sein, dass die Liegefläche durchbrochen ist und im Bereich dieses Durchbruchs die längenverschiebbare Magnetspule angeordnet ist, um einen möglichst dichten Kontakt der Magnetspule mit den Körperoberflächen der darauf liegenden Person zu ermöglichen.

Mit der Verwendung einer einzigen Magnetspule, die mit einem einzigen Generator zusammenarbeitet, ist eine kostengünstige Behandlung aller Körperteile einer auf der Behandlungsliege liegenden oder einer auf dem Behandlungsstuhl sitzenden Person möglich, weil erfindungsgemäß vorgesehen ist, dass die Magnetspule über den gesamten Behandlungsbereich der Behandlungsliege oder des Behandlungsstuhls verschiebbar ausgebildet ist.

Nach einem ersten Lösungsvorschlag der Erfindung ist es vorgesehen, dass die Längsverschiebung der Magnetspule mit einer drehend angetriebenen Spindelstange erfolgt, deren Längsachse parallel zur Mittenlängsachse der Liege oder des Stuhls ausgerichtet ist und die mindestens eine Spindelmutter durchgreift, die mit der Magnetspule verbunden ist.

Mit der Drehung der Spindelstange wird damit die Magnetspule, welche mit der Spindelmutter verbunden ist, entlang der Spindelstange in Längsrichtung der Liege oder des Stuhls bewegt, und auf diese Weise kann die Magnetspule an jede beliebige Stelle der Liege oder des Behandlungsstuhls verbracht werden.

In einer bevorzugten Ausgestaltung ist die Magnetspule in einer Kassette angeordnet, und die Spindelmutter ist an der Kassette angeordnet, so dass eine kompakte Anordnung gewährleistet ist.

In einer anderen Ausgestaltung der Erfindung kann es vorgesehen sein, dass die Längsverschiebung der Magnetspule (oder der Kassette, in der die Magnetspule angeordnet ist) mit einem in der Länge veränderbaren Zugseil erfolgt.

In einer dritten Ausgestaltung kann es vorgesehen sein, dass die Längsverschiebung der Magnetspule mit einem Antriebsmotor erfolgt, der in der die Magnetspule aufnehmenden Kassette angeordnet ist und der beidseitig angeordnete Ritzel antreibt, welche sich auf den mit Zahnstangen versehenen Führungsschienen an der Behandlungsliege abwälzen.

Bei allen Ausführungsformen ist es bevorzugt, wenn die Behandlungsliege aus mehreren Teilen besteht, die gegeneinander abklappbar sind. Somit kann die Behandlungsliege in einen Behandlungsstuhl und umgekehrt umgewandelt werden.

In einer bevorzugten Ausgestaltung bestehen die Behandlungsliege und/oder der Behandlungsstuhl aus mindestens einem schwenkbaren Kopfteil, das mit einem Mittelteil schwenkbar verbunden ist, wobei ein Mittelteil ein Fußteil schwenkbar ansetzt.

Erfindungsgemäß soll die verschiebbar angetriebene Magnetspule alle Teile dieser Behandlungsliege oder des Behandlungsstuhls erreichen und ist somit erfindungsgemäß sowohl in dem Kopfteil, als auch in dem Mittelteil, als auch in dem Fußteil verschiebbar angetrieben.

Die Erfindung ist nicht auf die Längsverschiebbarkeit einer oder mehrerer Magnetspulen beschränkt. Es kann in einer Weiterbildung vorgesehen sein, dass die Magnetspulen auch querverschiebbar und/oder drehbar ausgebildet sind.

Ebenso ist es in einer Weiterbildung vorgesehen, dass die eine oder mehreren Magnetspulen um eine horizontale Schwenkachse herum abklappbar ausgebildet sind.

Bei der Verwendung eines Magnetfeldapplikators in Form eines Behandlungsstuhls kann es durch eine geeignete Mechanik zusätzlich vorgesehen sein, dass beim Herunterfahren des Fußteiles der Behandlungsstuhl vorne automatisch hoch gefahren wird, damit das Fußteil länger gemacht werden kann.

Mit dem Gegenstand des unabhängigen Patentanspruches 1 wird erreicht, dass nun körperindividuell bestimmte Körperteile entsprechend den physiologischen Erfordernissen behandelt werden.

Beispielsweise wurde ermittelt, dass der Pelvis-Bereich und tiefergelegene Zielgebiete mit einer hohen Magnetfeldstärke und indikationsabhängig mit einer Frequenz zwischen 5 - 50 Hz behandelt werden sollten. Für den Rücken- und den Oberkörperbereich kann die Einstellung niedrigerer Intensitäten oder Frequenzen notwendig sein.

Die genannten Körperregionen (Bauch, Oberkörper, Nacken, Kopf oder auch kardiale Bereiche) werden deshalb mit geringeren Intensität oder Frequenz während des Verfahrweges der Magnetspule über oder an diesen Körperregionen vorbei behandelt, und ebenso wird es in einer Weiterbildung bevorzugt, wenn die Behandlung der einzelnen Körperteile nicht nur in Bezug auf die Frequenz verändert wird, sondern darüber hinaus auch noch auf die Magnetfeldstärke und/oder die Verweildauer der Magnetspule in diesem Körperbereich. Ebenso kann die Wiederholdauer, d. h. die Anzahl der Wiederholungen der Magnetbehandlung an der jeweiligen Körperstation und auch die Impulsform des verwendeten Magnetspulenstroms körperteil-individuell einstellbar ausgebildet sein.

Die jeweils verschiebbare Magnetspule ist mit einem Lagensensor verbunden, der die aktuelle Stellung und Position der Magnetspule im Bezug zur behandelten Körperregion der Person erfasst.

Das so erfasste Sensorsignal wird einem Generator eingegeben, der in seiner Steuerung die Amplitude des Magnetfeldes und/oder die Frequenz und/oder die Wiederholdauer und/oder die Behandlungszeit und/oder die Impulsform einstellen lässt.

Zu diesem Zweck wird es bevorzugt, wenn eine Programmierung des Generators mit einer Chipkarte erfolgt, auf der sämtliche Behandlungsdaten pro Körperteil eingespeichert sind, so dass mit dem genannten Programm eine körperteil-individuelle Behandlung während der automatischen Verschiebung der einen oder mehreren Magnetspulen längs der Behandlungsliege und/oder des Behandlungsstuhles erfolgen kann.

Als Beispiel für eine derartige körperteilindividuelle Behandlung dient die nachfolgende Tabelle:
Chipcard Programmierung: die Parameter sollen einzeln und frei programmierbar sein. So das der Therapeut die verschiedene Anforderungen auf die Chipkarte speichern kann.

| **Parameter** | **Einstellung** |
|---|---|
| Intensität | 20, 40, 60, 80, 100 % |
| Frequenz | 5 Hz bis 50 Hz |
| Behandlungsdauer | Von 5 min bis 60 min (teilbar bis 10 stufen) |
| Pulsation Dauer | Von 1 s bis 100 s |
| Pulsation Pause | Von 1 s bis 300 s |
| Pulsation Wiederholung | Von 1 bis 10 |
| Spulenposition | Von 0 bis 15 cm |
| | |
| | |

### Programm Beispiel:

**Tabelle1 :Beispiel für die körperteil-individuelle Behandlung einer Person mit an dem Körperteil angepassten Magnetfeldern.**

| Programm + Spulenposition | Frequenz | Pulsation | Gesamtzeit |
|---|---|---|---|
| Programm 1 Muskeltonusprogramm | Frequenz 20 Hz | 4 Sek. ein / 8 Sek. aus 2 Wiederholungen x 12 Sek. | 24 Sek |
| Typ 1-Fasem / oxidativaerob / | Frequenz 21 Hz | 4 Sek. ein / 8 Sek. aus 2 Wiederholungen x 12 Sek. | 24 Sek |
| Spulen Position (0 bis 7 cm) | Frequenz 22 Hz | 4 Sek. ein / 8 Sek. aus 2 Wiederholungen x 12 Sek. | 24 Sek |
| | Frequenz 23 Hz | 4 Sek. ein / 8 Sek. aus 2 Wiederholungen x 12 Sek. | 24 Sek. |
| | Frequenz 24 Hz | 4 Sek. ein / 8 Sek. aus 2 Wiederholungen x 12 Sek. | 24 Sek |
| | Frequenz 25 Hz | 4 Sek. ein / 8 Sek. aus 2 Wiederholungen x 12 Sek. | 24 Sek. |
| | | | 120 Sek. |
| Spulen Position (0 bis 7 cm) | Frequenz 20 Hz | 4 Sek. ein / 8 Sek. aus 2 Wiederholungen x 12 Sek. | 24 Sek |
| | Frequenz 21 Hz | 4 Sek. ein / 8 Sek. aus 2 Wiederholungen x 12 Sek. | 24 Sek |
| | Frequenz 22 Hz | 4 Sek. ein / 8 Sek. aus 2 Wiederholungen x 12 Sek. | 24 Sek |
| | Frequenz 23 Hz | 4 Sek, ein / 8 Sek. aus 2 Wiederholungen x 12 Sek. | 24 Sek |
| | Frequenz 24 Hz | 4 Sek. ein / 8 Sek. aus 2 Wiederholungen x 12 Sek. | 24 Sek |
| | Frequenz 25 Hz | 4 Sek. ein / 8 Sek. aus 2 Wiederholungen x 12 Sek. | 24 Sek |
| | | | 120 Sek |
| Spulenposition (7-15 cm) | Frequenz 20 Hz | 4 Sek. ein / 8 Sek. aus 2 Wiederholungen x 12 Sek. | 24 Sek |
| | Frequenz 21 Hz | 4 Sek. ein / 8 Sek. aus 2 Wiederholungen x 12 Sek. | 24 Sek |
| | Frequenz 22 Hz | 4 Sek. ein / 8 Sek. aus 2 Wiederholungen x 12 Sek. | 24 Sek |
| | Frequenz 23 Hz | 4 Sek. ein / 8 Sek. aus 2 Wiederholungen x 12 Sek. | 24 Sek |
| | Frequenz 24 Hz | 4 Sek. ein / 8 Sek. aus 2 Wiederholungen x 12 Sek. | 24 Sek |
| | Frequenz 25 Hz | 4 Sek. ein / 8 Sek. aus 2 Wiederholungen x 12 Sek. | 24 Sek |
| | | | 120 Sek |
| Spulen position (7-15 cm) | Frequenz 20 Hz | 4 Sek. ein / 8 Sek. aus 2 Wiederholungen x 12 Sek, | 24 Sek |
| | Frequenz 21 Hz | 4 Sek. ein / 8 Sek. aus 2 Wiederholungen x 12 Sek. | 24 Sek |
| | Frequenz 22 Hz | 4 Sek. ein / 8 Sek. aus 2 Wiederholungen x 12 Sek. | 24 Sek |
| | Frequenz 23 Hz | 4 Sek. ein / 8 Sek. aus 2 Wiederholungen x 12 Sek. | 24 Sek |
| | Frequenz 24 Hz | 4 Sek. ein / 8 Sek. aus 2 Wiederholungen x 12 Sek. | 24 Sek |
| | Frequenz 25 Hz | 4 Sek. ein / 8 Sek. aus 2 Wiederholungen x 12 Sek. | 24 Sek |
| | | | 120 Sek |
| | Pause | Pause | 180 Sek |
| | Wiederholung | Wiederholung | 480 Sek |
| | Gesamtzeit | Gesamtzeit | 1 140 Sek |
| | | | |

Diese Tabelle ist die Vorlage für eine körperteil-individuelle Programmierung eines Generators, der die mindestens eine Magnetspule ansteuert.

### Programm Einstellung

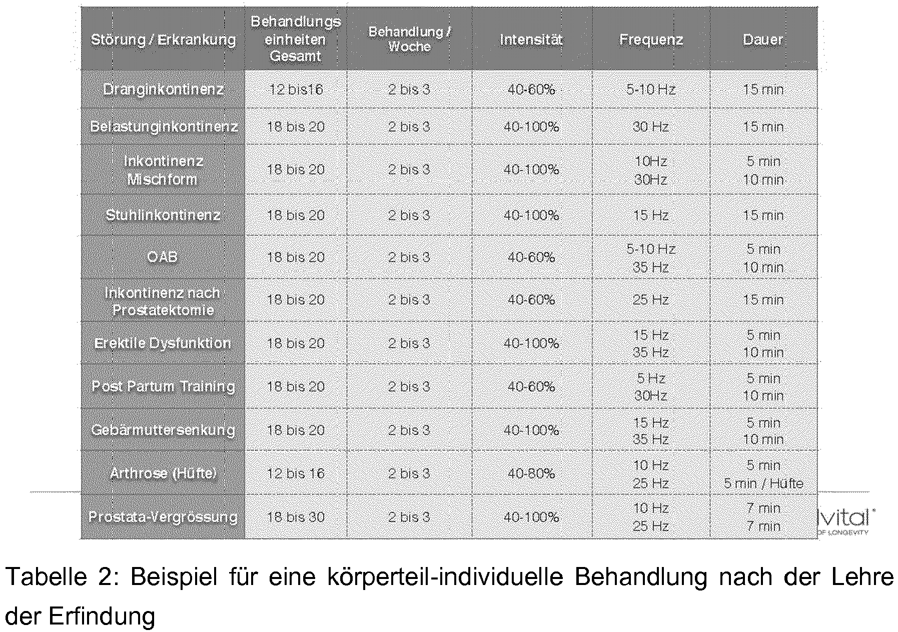

Die Tabelle 2 zeigt Beispiele für die körperteil-individuelle Behandlung in Abhängigkeit von der medizinischen Indikation mittels der einen oder der mehreren verschiebbar angeordneten Magnetspulen. Die programmierte automatische Verschiebung der Magnetspule(n) erfolgt an den Ort des zu behandelnden Körperteils oder des zu behandelnden Organs.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Die anderen Ausführungsformen sowie Behandlungsverfahren sind kein Gegenstand der Erfindung.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: schematisiert die Seitenansicht einer ersten Ausführungsform eines Magnetfeldapplikators in der Art einer Behandlungsliege
- Figur 2:: schematisiert die Seitenansicht der Behandlungsliege nach Figur 1 mit Darstellung weiterer Einzelheiten
- Figur 3:: die Draufsicht auf die Behandlungsliege nach Figur 2
- Figur 4:: der Schnitt durch die Behandlungsliege nach IV-IV nach Figur 2 und Figur 3
- Figur 5:: eine gegenüber Figur 4 abgewandelte Ausführungsform
- Figur 6:: eine weitere gegenüber Figur 4 und 5 abgewandelte Ausführungsform
- Figur 7:: eine vierte Ausführungsform einer Behandlungsliege oder eines Behandlungsstuhls im Schnitt gemäß VII-VII in Figur 8
- Figur 8:: die Draufsicht auf die Anordnung nach Figur 7
- Figur 9-11:: die Darstellung der verschiedenen Schwenkpositionen einer Behandlungsliege oder eines Behandlungsstuhls mit unterschiedlichen Verschiebungsorten der Magnetspule.
- Figur 12-15:: piktogramm-artige Darstellung der verschiedenen Verschiebepositionen der Spule in Verbindung mit verschiedenen Schwenklagen der Behandlungsliege
- Figur 16:: eine beispielhafte Darstellung einer Vorrichtung mit zwei verschiebbaren Spulen
- Figur 17:: das Frequenz-Verschiebungsdiagramm, was zeigt, an welchen Körperregionen im Bereich der Rückenspule welche Frequenzen vorteilhaft angewendet werden
- Figur 18:: das Frequenz-Verschiebungsdiagramm der horizontal verschiebbaren Spule
- Figur 19:: ein Diagramm zur Darstellung der ortsveränderlichen Leistung des Magnetfeldes in Abhängigkeit von der Verschiebungslänge in Bezug zu verschiedenen Organen und Körperteilen
- Figur 20:: die technische Ausführung einer Behandlungsvorrichtung, die lediglich schematisiert in Figur 16 dargestellt ist

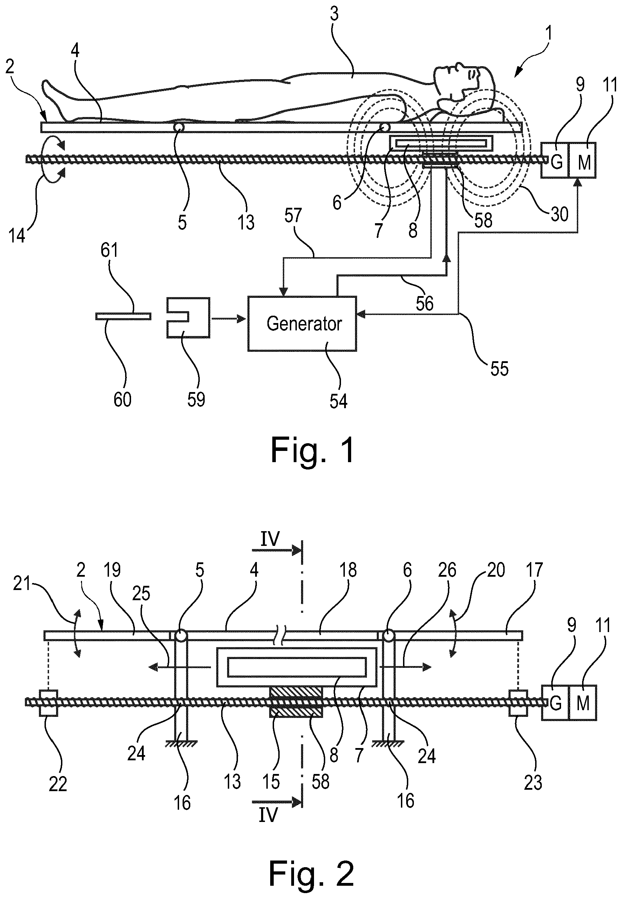

In Figur 1 und 2 besteht der Magnetfeldapplikator 1 im Wesentlichen aus einer Behandlungsliege 2, die etwa rechteckförmig und gestellartig ausgebildet ist und mit einer Anzahl von Stützen 16 auf einem Aufstellungsort abgestützt ist.

Die Liegefläche 4 wird durch einen oberen Rahmen gebildet, auf den noch eine Auflage aufgelegt sein kann, auf der eine zu behandelnde Person 3 aufliegt.

Die Behandlungsliege 2 besteht aus mehreren voneinander abklappbaren Teilen, wobei die Liegefläche 4 durch Gelenke 5, 6 abklappbar ist.

Somit ist der Kopfteil 17 von dem Mittelteil 18 abklappbar, und der Fußteil 19 ist vom Mittelteil 18 abklappbar. Die genannten Teile sind in den Pfeilrichtungen 20, 21 bewegbar. Die Behandlungsliege 2 kann damit in einen Behandlungsstuhl umgewandelt werden.

An der Unterseite des Rahmens, welcher die Liegefläche 4 definiert, ist eine Kassette 7 angeordnet, in der eine Magnetspule 8 angeordnet ist.

Als Beispiel ist angegeben, dass der Magnetspule ein den Körper der Person durchsetzendes Magnetfeld 30 erzeugt.

Der längsverschiebbare Antrieb der Kassette 7 erfolgt dadurch, dass die Kassette 7 an ihrer Unterseite mit einer Spindelmutter 15 verbunden ist, die von einer drehend angetriebenen Spindelstange 13 durchgriffen ist.

Das eine Ende der Spindelstange 13 ist mit einem Getriebe 9 drehfest verbunden, das von einem Motor 11 drehend angetrieben ist.

Auf diese Weise ist die Spindelstange 13 in Drehrichtung vorwärts- und rückwärtsgehend angetrieben, so dass bei einem Antrieb der Spindelstange die Kassette 7 in den Pfeilrichtungen 25, 26 unterhalb der Liegefläche 4 der Behandlungsliege 2 bewegt wird.

In Figur 1 ist eine Steuervorrichtung für den Magnetfeldapplikator 1 dargestellt, die lagen- und körperteilabhängig die Art und Funktion des Magnetfeldes positionsabhängig steuert.

Zu diesem Zweck ist die Magnetspule 8 mit einem Sensor 58 verbunden, der in der Lage ist, die aktuelle Position der Magnetspule längs der Behandlungsliege 2 zu erfassen. Das Signal des Sensors 58 wird über eine Sensorleitung 57 einem Generator 54 eingespeist, welcher das Magnetfeld der Magnetspule 8 erzeugt. Der Sensor kann jedoch die Umdrehung der Spindel erfassen, um so die aktuelle Position der Magnetspule längs der Behandlungsliege zu erfassen. Die Verschiebung der Spule längs der Behandlungsliege kann als Lagenregelung ausgebildet sein, das heißt, dass die Umdrehungszahl und die Start/Stopp-Position des Antriebsmotors in Abhängigkeit von der Lage der Magnetspule regelbar ist.

Der Generator 54 ist über eine Chipkarte 60 mit einem darauf angeordneten EEPROM 61 programmierbar, so dass entsprechend der Lage und der Position der Magnetspule 8 körperteilabhängig das Magnetfeld modifiziert wird. Statt einer programmierten Chipkarte kann auch jeder andere Datenspeicher zur Programmierung verwendet werden.

Zum programmierten Betrieb des Generators werden die zur Versorgung der Magnetspule 8 notwendigen Ströme über die Stromleitung 56 vom Generator 54 modifiziert. Gleichfalls wirkt auf den Generator 54 eine Steuerleitung 55, die mit dem Motor 11 in Verbindung steht, um in Abhängigkeit von der körperteilbezogenen Stellung der Magnetspule die Verweildauer der Magnetspule an dieser Stelle und/oder die Wiederholfrequenz der Behandlung an dieser Stelle zu steuern.

Die Chipkarte 60 wird im Übrigen von einem Kartenleser 59 erfasst.

Statt einer Programmierung auf einer Chipkarte 60 können auch alle anderen Programmierarten und Datenübertragungen für das Einlesen der Behandlungsdaten in den Generator 54 verwendet werden, wie z. B. eine WLAN-Anbindung oder eine LAN-Anbindung des Generators 54 an zugeordnete Informationsquellen, die auch in einer Cloud oder auf einem Internet-Server liegen können.

Gemäß Figur 2 kann die Spindelstange 13 noch in Lagern 23 abgestützt sein, wobei die Lager 22, 23 an beiden Enden der Spindelstange 13 angeordnet sind. Das eine Lager 22 kann mit der Unterseite des Fußteils 19 und das andere Lager 23 mit dem Kopfteil 17 verbunden sein.

Die Spindelstange 13 kann abklappbar ausgebildet sein und fluchtend gegenüber den Gelenken 5, 6 der Behandlungsliege 12 Knickstellen 24 aufweisen. Dies ermöglicht bei einem in Pfeilrichtung 20 abgeklappten Kopfteil 17, dass der Teil der Spindelstange 13 zwischen dem Lager 23 und der Knickstelle 24 ebenfalls abgeknickt wird..

Die Abknickung sollte jedoch erst dann erfolgen, wenn die Kassette 7 durch den Drehantrieb der Spindelstange 13 in Pfeilrichtung 14 in den gewünschten Bereich des Kopfteils 17 befördert wurde.

Gleiches gilt auch für den Verschiebeantrieb der Kassette 7 in den Bereich des Fußteils 19. Auch dort sollte das Fußteil 19 erst dann in Pfeilrichtung 21 hochgeklappt werden, wenn der Verschiebeantrieb die Kassette 7 in den Bereich des Fußteils 19 in Pfeilrichtung 25 verbracht hat.

Die Ausführungsform nach Figur 1 und 2 beschreibt demnach einen Magnetfeldapplikator 1 mit einer unten liegenden Spindelmutter 15 und einer unten liegenden dazugehörenden Spindelstange 13, die entweder durchgehend oder abknickbar ausgebildet sein kann.

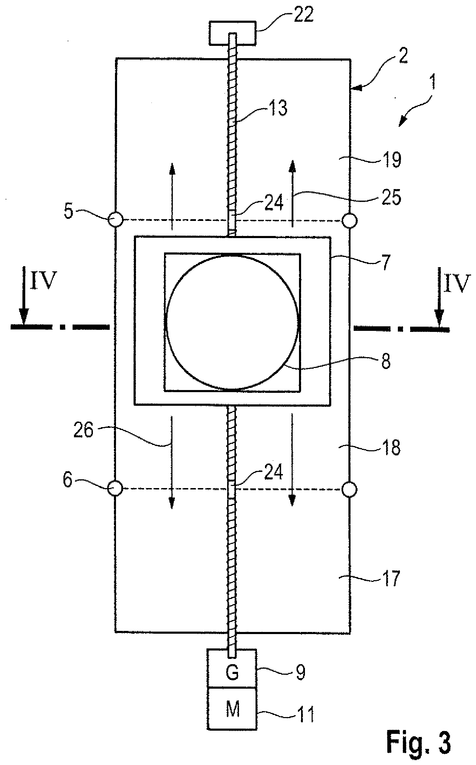

Eine solche Ausführung ist in Draufsicht in Figur 3 dargestellt. Dort ist erkennbar, dass die Magnetspule 8 etwa zylinderförmig ausgebildet ist und in einer rechteckförmigen Kassette 7 angeordnet ist.

Die Kassette 7 kann noch über zusätzliche Führungsglieder an der Unterseite des Rahmens der Behandlungsliege 2 verfügen.

Ansonsten gelten für die gleichen Teile die gleichen Bezugszeichen.

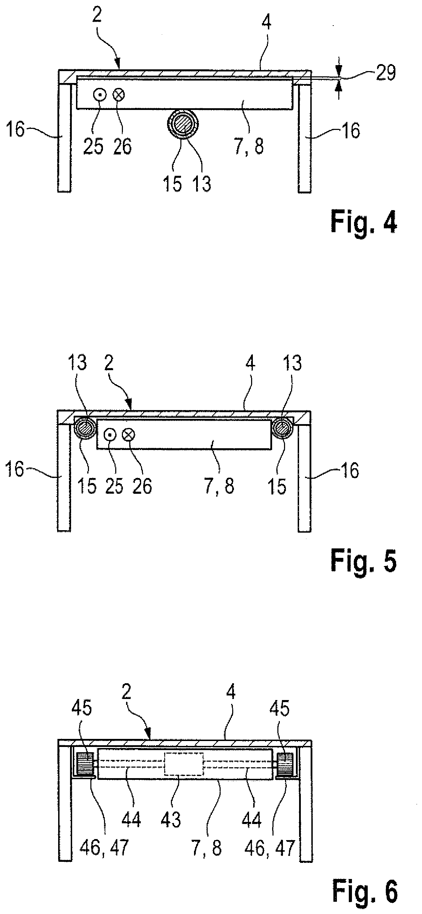

Die Figur 4 zeigt die gleiche Anordnung nach Figur 1 bis 3 im Schnitt gemäß der Linie IV-IV, und es ist erkennbar, dass die Oberkante der Kassette 7 mit der ebenfalls - dort oberkantenbündig eingebauten - Magnetspule 8 einen geringen Abstand 29 zur Liegefiäche 4 aufweist. Der Abstand 29 kann bis auf 0 gehen.

Ebenso ist dargestellt, dass eine einzige, mittige Spindelstange 13 die mittig unterhalb der Kassette 7 angeordnete Spindelmutter 15 durchgreift.

In Abweichung vom Ausführungsbeispiel nach Figur 4 kann jedoch vorgesehen sein, dass zwei zueinander parallele Spindelstangen 13 mit zwei jeweils zugeordneten Spindelmuttern 15 vorgesehen sind, um einen kompakteren Aufbau zu erreichen. Damit wird die Bauhöhe der Behandlungsliege vermindert.

Die Figur 6 zeigt als weiteres Ausführungsbeispiel im Vergleich zu den den Figuren 1 bis 5, dass es auch möglich ist, einen autonomen Fahrantrieb in der Kassette 7 anzuordnen. Dabei ist in der Kassette 7 ein Akku angeordnet, der den dort angeordneten Antriebsmotor 43 mit Strom versorgt. Dieser treibt über die Antriebswellen 44 beidseits drehfest mit den Antriebswellen 44 verbundene Ritzel 45 an, die auf zugeordneten Zahnstangen 46 abrollen, die im Bereich einer L-förmig profilierten Führungsschiene 47 angeordnet sind.

Auf diese Weise kann auf Zugmittel verzichtet werden, weil der Kassette 7 ein autonomer, selbstfahrender Antrieb zugeordnet ist.

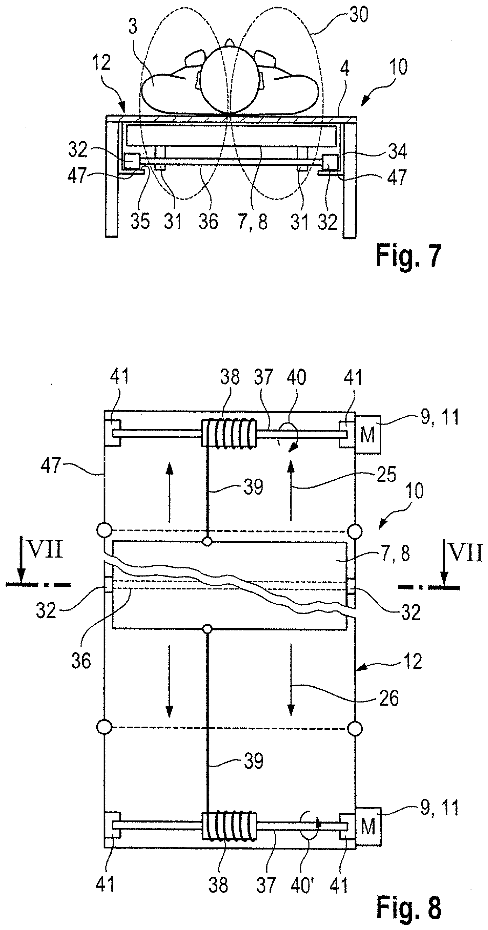

Die Figur 7 zeigt ein von der Figur 6 abgewandeltes Ausführungsbeispiel und die Figur 7 betrifft jedoch auch ein Ausführungsbeispiel, welches in Figur 8 in Draufsicht dargestellt ist.

Dort ist erkennbar, dass an der die Magnetspule 8 aufnehmenden Kassette 7 jeweils Zugseile 39 angeordnet sind und jedes Zugseil auf einander gegenüberliegende Wickeltrommeln 38 aufgewickelt ist. Jede Wickeltrommel 38 ist drehfest mit einer zugeordneten Wickelwelle 37 verbunden, die jeweils ineinander gegenüberliegenden Lagern 41 unterhalb der Liegefläche 4 gelagert sind.

Jede der Wickelwellen 37 wird von einer Motorgetriebeanordnung 9, 11 drehend angetrieben.

Somit erfolgt eine Bewegung der Kassette 7 in Pfeilrichtung 25, wenn die obere Wickelwelle 37 in Pfeilrichtung 40 angetrieben ist, wickelt sich die untere Wickelwelle 37 in Pfeilrichtung 40' ab.

Anstatt der Anordnung von zwei Motor-Getriebeanordnungen 9, 11 kann es auch vorgesehen sein, dass lediglich eine einzige Motor-Getriebeanordnung 9, 11 vorhanden ist und die andere Motor-Getriebeanordnung durch eine Federanordnung ersetzt ist. Eine solche Federanordnung ist z.B. ein elastisches Zugseil, was mehr oder weniger eine mechanische Zugkraft auf die der Motor-Getriebeanordnung gegenüber liegenden Seite der Kassette ausübt. Auf diese Weise wird das Zugseil 39 auf der einen Seite stets unter der Federspannung gehalten und der Motor 11 an der gegenüberliegenden Seite kann die Kassette 7 mit der dort angeordneten Magnetspule 8 frei über die gesamte Länge des Magnetfeldapplikators 10 verschieben.

Es ist auch noch dargestellt, dass die Kassette 7 über Stützen 31 auf einer Welle 36 abgestützt ist und die Welle beidseitig Rollen 32 trägt, die auf zugeordneten Führungsflächen einer Führungsschiene 47 der Behandlungsliege 10 abrollen.

Die Laufflächen 35 sind somit im Bereich der Stützwinkel 34 als horizontale Flächen ausgebildet.

Wie eingangs ausgeführt, kann eine solche Führungsanordnung auch in Verbindung mit dem autonomen Fahrantrieb der Kassette 7 nach dem Ausführungsbeispiel nach Figur 6 verwendet werden.

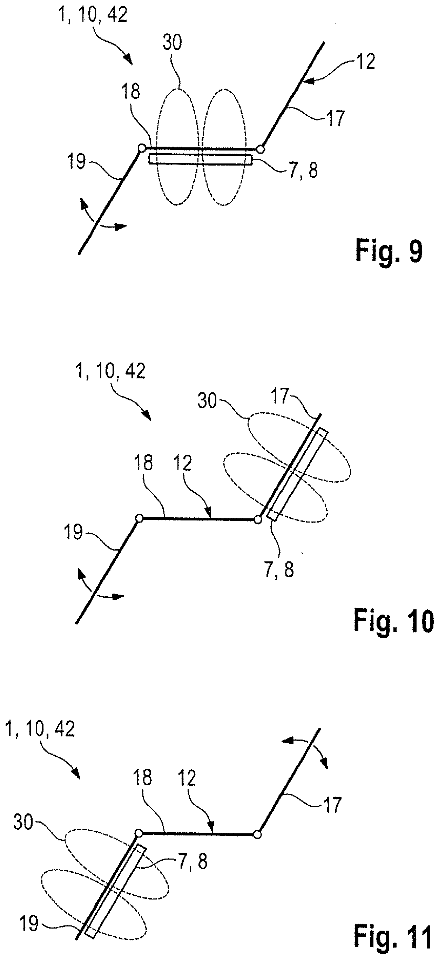

Die Figuren 9 bis 11 zeigen verschiedene Stellungen eines Magnetfeldapplikators 1, 10 oder eines Behandlungsstuhls 42 in verschiedenen Behandlungssituationen.

In der Behandlungssituation nach Figur 9 ist die Kassette 7 mit der dort angeordneten Magnetspule 8 unterhalb des Mittelteils 18 verfahren und eine darauf sitzende Person wird mit dem mittleren Körperteil von dem Magnetfeld 30 durchsetzt, während das Kopfteil 17 und das Fußteil 19 abgeklappt sind.

Bei einer Längsverschiebung der Kassette 7 mit der Magnetspule 8 gemäß Figur 10 wird diese in den Bereich des Kopfteils 17 gebracht und das sich dort entwickelnde Magnetfeld 30 durchsetzt den Oberkörperbereich der Person 3.

In analoger Weise kann aufgrund des längsverschiebbaren Antriebes der Kassette 7 mit der dort angeordneten Magnetspule 8 diese auch in den Bereich des Fußteils 19 verfahren werden, wobei dann das Magnetfeld 30 den Unterkörperbereich und den Beinbereich der Person 3 behandelt.

In der vorstehenden Beschreibung wurde lediglich eine Längsverschiebung der Kassette 7 mit einer daran angebauten Magnetspule 8 längs des Magnetfeldapplikators 1, 10, 42 beschrieben.

In einer Weiterbildung der Erfindung kann es auch vorgesehen sein, dass die Kassette 7 mit der dort eingebauten Magnetspule 8 kleiner ist als die Breite des Magnetfeldapplikators 1, 10, 42, und in diesem Fall ist es vorgesehen, dass nicht nur eine Längsverschiebung des Magnetfeldapplikators 1, 10, 42 gegeben ist, sondern auch eine Querverschiebung, d. h. senkrecht zur Längsmittenachse durch den Magnetfeldapplikator 1, 10, 42.

Ebenso kann es vorgesehen sein, die Magnetspule und/oder die Kassette, welche die Magnetspule aufnimmt, um eine vertikale Achse herum drehbar zu gestalten.

Es wurde bei der Durchführung von Doppelblindstudien festgestellt, dass Personen, die mit dem Magnetfeldapplikator 1, 10, 42 behandelt werden, sich vom Geräusch des Magnetfeldapplikators beeinflussen lassen und davon ausgehen, dass aufgrund des akustisch wahrnehmbaren Geräusches auch eine Erzeugung eines Magnetfeldes 30 gegeben ist, die den Körper der Person 3 durchsetzt.

Um derartige Doppelblindstudien objektiv zu gestalten, kann es vorgesehen sein, dass die Magnetspule 8 und/oder die Kassette 7 mit der dort enthaltenen Magnetspule 8 von der Behandlungsliege 2, 12 oder vom Behandlungsstuhl 42 um eine horizontale Achse nach unten abklappbar ausgestaltet ist, was bedeutet, dass das dann erzeugte Magnetfeld 30 nicht mehr senkrecht die Liegefläche 4 des Magnetfeldapplikators 1, 10, 42 durchsetzt, sondern völlig unwirksam unterhalb des Körpers der Person parallel zur Längsachse der Behandlungsliege entsteht. Damit hört die Person zwar eine elektrische Aktivität der Magnetspule, es ist aber sichergestellt, dass in diesem Fall das Magnetfeld 30 nicht mehr den Körper der Person 3 durchsetzt, wodurch objektive Doppelblindstudien möglich sind und die zu behandelnde Person 3 nicht mehr annehmen muss, dass der Körper auch bei Wahrnehmung eines Geräusches vom Magnetfeld 30 durchsetzt wird.

Die Abklappbarkeit der Magnetspule 8 bedeutet, dass dann das sich entwickelnde Magnetfeld mit seiner Längsrichtung parallel zur Längsachse der Behandlungsliege 2, 12 ausgerichtet ist.

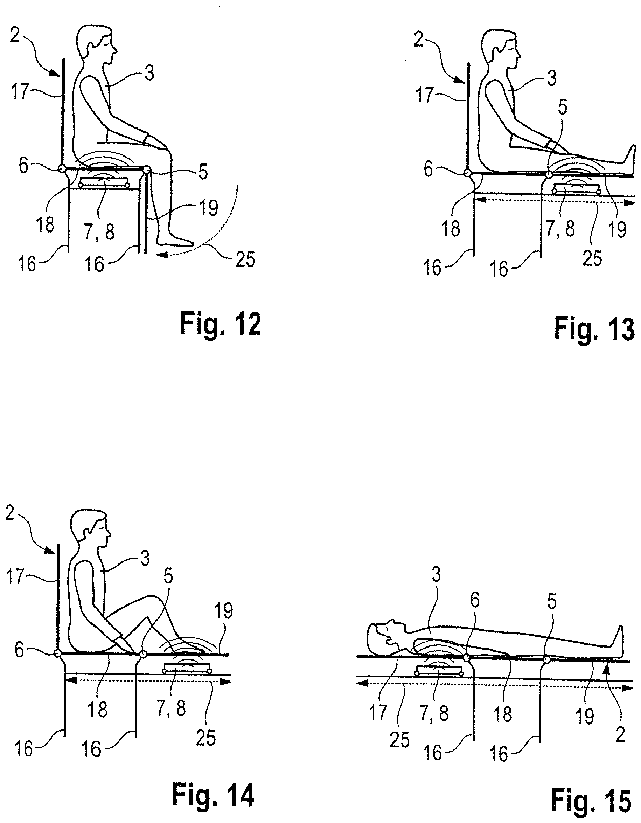

Die Figuren 12 bis 15 zeigen in piktogrammartiger Darstellung die verschiedenen Behandlungspositionen und Stellungen der Behandlungsliege 2.

In Figur 12 ist dargestellt, dass die Behandlungsliege 2 als Stuhl verwendet wird, wobei das Kopfteil 17 hochgestellt ist, das Mittelteil 18 die Sitzfläche bildet und das Fußteil 19 abgeklappt ist.

Die Kassette 7 mit der eingebauten Magnetspule 8 ist deshalb lediglich im Bereich der Sitzfläche des Mittelteils 18 angeordnet.

Es kann vorgesehen sein, dass die Kassette 7 dort unverschiebbar gehalten ist. Es kann jedoch in einer anderen Ausgestaltung vorgesehen sein, dass die Kassette 7 mit der eingebauten Magnetspule 8 sowohl in Längsrichtung der Sitzfiäche, d. h. im Zwischenraum zwischen den Gelenken 5 und 6 verschiebbar ist, jedoch auch in Querrichtung.

In Figur 13 ist eine weitere Behandlungsmöglichkeit dargestellt, bei der im Vergleich zur Figur 12 das Fußteil 19 hochgeklappt ist.

In diesem Fall ist es möglich, die Kassette 7 mit der eingebauten Magnetspule 8 sowohl im Bereich zwischen dem fußseitigen Gelenk 5 und dem kopfseitigen Gelenk 6 zu verschieben, aber jedoch auch noch darüber hinaus in Pfeilrichtung 25 bis zum Ende des Fußteils 19.

Auch in diesem Fall kann somit die Kassette 7 im eingezeichneten Bereich der Pfeilrichtung 15 über die gesamte Länge des Mittelteils 18 und des Kopfteils 19 verschoben werden, jedoch auch in Querrichtung hierzu.

Die Figur 14 zeigt im Vergleich zur Figur 13 eine andere Behandlungsposition der Person 3, wo erkennbar ist, dass die Person 3 auch mit angezogenen Beinen auf der Behandlungsliege 2 sitzen kann, wie in Figur 14 dargestellt. In diesem Fall werden die Füße von der Magnetspule 8 in der Kassette 7 mit einem Magnetfeld beaufschlagt. Die Kassette 7 kann jedoch auch im eingezeichneten Bereich der Pfeilrichtung 15 bis in die Nähe des kopfseitigen Gelenks 6 verschoben werden und auch in Querrichtung hierzu.

Die Figur 15 zeigt die Stellung der Behandlungsliege 2, wie sie auch in den Figuren 1 und 2 dargestellt ist. In diesem Ausführungsbeispiel kann somit die Kassette 7 mit der eingebauten Magnetspule 8 in den Pfeilrichtungen 25 im vollen eingezeichneten Bereich sowohl über das Kopfteil 17, das Mittelteil 18 und das Fußteil 19 verschoben werden. Auch eine Querverschiebung - senkrecht zur Zeichenebene der Figuren 12 bis 15 - ist möglich.

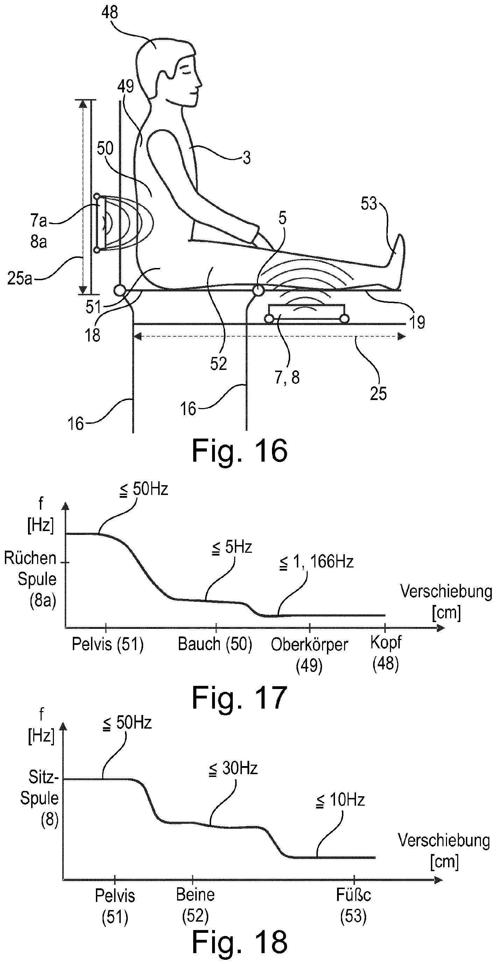

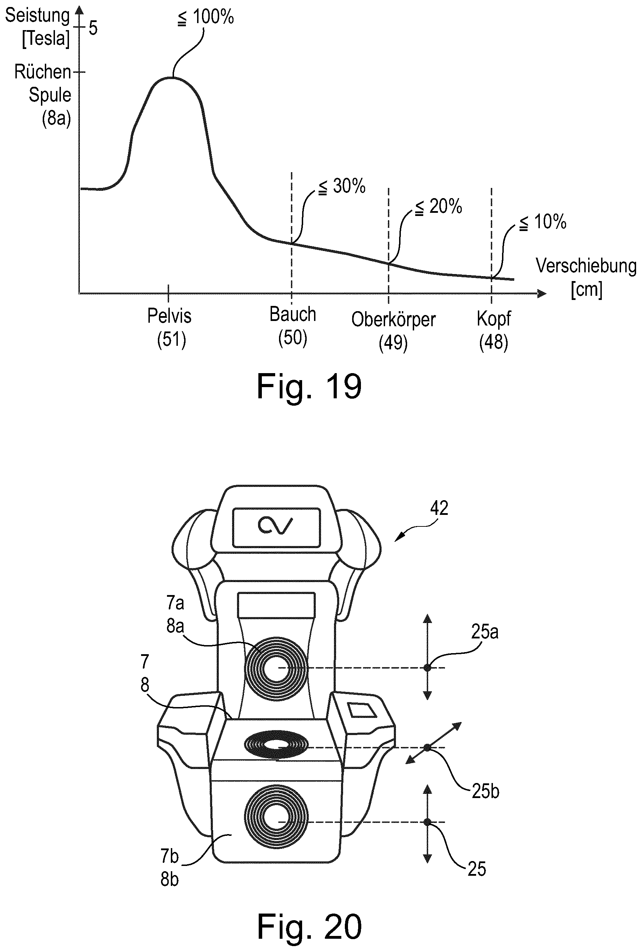

In Figur 16 ist schematisiert der Aufbau und die Funktion eines Behandlungsstuhls 42 nach Figur 20 dargestellt.

Die oberkörperseitigen Körperteile der Person 3, wie z. B. der Kopf 48, der Oberkörper 49 und der Bauch 50 sowie der Pelvis 51 werden von der rückenseitigen Magnetspule 8a behandelt, die - wie vorher beschrieben - vorteilhaft in einer zugeordneten Kassette 7a angeordnet ist, so dass die gesamte Anordnung in den Pfeilrichtungen 25a in vertikaler Richtung entlang der Rückenlehne verschiebbar ist.

In analoger Weise gilt dies für die dem Sitz und dem ausklappbaren Fußteil zugeordnete Magnetspule 8, die in den Pfeilrichtungen 25 längs verschiebbar angetrieben ist und in diesem Fall dem Pelvis 51, die Beine 52 und die Füße 53 einer Magnetbehandlung unterwerfen kann.

Es ist ebenfalls nicht dargestellt, dass während der Magnetfeldbehandlung oder vor der Magnetfeldbehandlung noch zusätzlich Sauerstoff in die Atemluft der Person zugeführt werden kann.

Dadurch wird die Sauerstoffaufnahme im Gewebe verbessert und der Therapieerfolg ist verstärkt.

Gleichfalls ist zeichnerisch nicht dargestellt, dass die Person während des Betriebs des Magnetfeldapplikators 1, 10 muskuläre Körperarbeit leisten kann und dass zu diesem Zweck muskuläre Trainingsgeräte an der Behandlungsliege 2, 12 und/oder am Behandlungsstuhl 42 angeordnet sind. Derartige muskuläre Trainingsgeräte können ein oder mehrere Gummi sein, Expander, federbelastete Schwenkhebel, Hebegewichte und dergleichen sein.

Die Figur 17 zeigt als Ausführungsbeispiel, dass die Frequenz der Rückenspule in Abhängigkeit von der behandelten Körperregion von dem Generator 54 variiert wird.

Es wurde festgestellt, dass der Bereich des Pelvis 51 oder auch der sonstigen Skelettmuskulatur, je nach Inkontinenzart oder Muskelfasertypus, mit einer Frequenz zwischen 5 - 50 Hz magnetisiert werden sollten, während vom Pelvis aufwärts oder auch in der Wirbelsäulen-, Herz- oder Kopfregion niedrigere Intensitäten und/oder auch Frequenzen (1,166 Hz - 50 Hz) verwendet werden.

Das Diagramm in Figur 18 zeigt die Frequenzkurve in der Abhängigkeit von der Verschiebungslage der in horizontaler Pfeilrichtung 25 verschiebbaren Magnetspule 7, wo ebenfalls erkennbar ist, dass die Pelvis-Region mit einer hohen Intensität und einer Frequenz von bis zu 50 Hz behandelt werden sollte, während die die untere Extremität je nach Lage und Tiefe der Muskulatur nur einer geringeren Intensität bedarf.

Die Figur 19 zeigt auch, dass die Magnetfeldstärke, die in Tesla ausgedrückt wird, im Bereich des Pelvis bis zu 5 Tesla betragen kann und damit einer etwa 100% Leistung des Generators 54 entspricht.

Der Oberkörper sollte mit einer verringerten Magnetfeidieistung von weniger als 20 % behandelt werden.

Weitere Diagramme, weiche die Wiederholfrequenz der Magnetbehandlung für jeden angegebenen Körperteil darstellen, sind zeichnerisch nicht dargestellt. Auch hier gilt, dass die Wiederholfrequenz der Behandlung und auch die Behandlungsdauer körperteilabhängig vom Generator 54 gesteuert werden.

Ebenso gilt dies für die Impulsform der Magnetströme, die der jeweiligen Magnetspule 8, 8a, 8b zugeführt werden, wobei vorteilhaft jede Magnetspule in eine Kassette 7, 7a, 7b gefasst ist.

So zeigt die Figur 20, dass die in der Rückenlehne angeordnete Magnetspule 8a in den Pfeilrichtungen 25a längs der Rückenlehne verschiebbar ist, während die in der Sitzfläche angeordnete Magnetspule 8 längs der Sitzfläche in Pfeilrichtung 25b verschiebbar ist und die im abklappbaren Fußteil angeordnete weitere Magnetspule 8b ebenfalls in Pfeilrichtung 25 verschiebbar ist.

Mit der gegebenen technischen Lehre wird demnach ein Verfahren beschrieben, mit dem körperteilabhängig eine Magnetfeldbehandlung mit unterschiedlichen Magnetfeldapplikatoren 1, 10 durchgeführt werden kann, wobei bevorzugt eine automatisierte Behandlung mittels eines programmierbaren Generators 54 erfolgt.

Dementsprechend erfolgt eine programmierte Verschiebung der Magnetspulen 8, 8a, 8b in Abhängigkeit der oben genannten Parameter.

### Zeichnunaslegende

- 1: Magnetfeldapplikator
- 2: Behandlungsliege
- 3: Person
- 4: Liegefläche
- 5: Gelenk (Fuß-)
- 6: Gelenk (Kopf-)
- 7: Kassette 7a, 7b
- 8: Magnetspule 8a, 8b
- 9: Getriebe
- 10: Magnetfeldapplikator
- 11: Motor
- 12: Behandlungsliege
- 13: Spindelstange
- 14: Pfeilrichtung
- 15: Spindelmutter
- 16: Stütze
- 17: Kopfteil
- 18: Mittelteil
- 19: Fußteil
- 20: Pfeilrichtung
- 21: Pfeilrichtung
- 22: Lager
- 23: Lager
- 24: Knickstelle
- 25: Pfeilrichtung 25a, 25b
- 26: Pfeilrichtung
- 27: Spindelmutter
- 28: Spindelstange
- 29: Abstand
- 30: Magnetfeld
- 31: Stütze
- 32: Rolle
- 34: Stützwinkel
- 35: Lauffläche
- 36: Welle
- 37: Wickelwelle
- 38: Wickeltrommel
- 39: Zugseil
- 40: Pfeilrichtung 40'
- 41: Lager
- 42: Behandlungsstuhl
- 43: Antriebsmotor
- 44: Antriebswelle
- 45: Ritzel
- 46: Zahnstange
- 47: Führungsschiene
- 48: Kopf
- 49: Oberkörper
- 50: Bauch
- 51: Pelvis
- 52: Beine
- 53: Füße
- 54: Generator
- 55: Steuerleitung
- 56: Stromleitung (für 8)
- 57: Sensorleitung
- 58: Positionssensor
- 59: Kartenleser
- 60: Chipkarte
- 61: EEPROM

## Patentansprüche

1. Vorrichtung zum Betrieb eines Magnetfeldapplikators (1, 10) zur Magnetfeldbehandlung von Personen (3) auf einer Behandlungsliege (2, 12) und/oder einem Behandlungsstuhl (42) mit mindestens einer Magnetspule (8, 8a, 8b), wobei die mindestens eine Magnetspule (8, 8a, 8b) mindestens teilweise über die Länge der Behandlungsliege (2, 12) und/oder des Behandlungsstuhls (42) längsverschiebbar ausgebildet ist und ein den Körper der Person (3) mindestens teilweise durchdringendes Magnetfeld (30) erzeugbar ist, **dadurch gekennzeichnet, dass** während der Längsverschiebung der mindestens einen Magnetspule (8, 8a, 8b) die Stärke des mindestens einen Magnetfeldes (30) und/oder dessen Frequenz positionsabhängig in Abhängigkeit von der Region des Körpers der Person (3) steuerbar ist, wobei die verschiebbare Magnetspule (8, 8a, 8b) mit einem Positionssensor (58) verbunden ist, der die aktuelle Stellung und Position der Magnetspule (8, 8a, 8b) in Bezug zur behandelten Körperregion der Person (3) erfasst, und das so erfasste Sensorsignal einem Generator (54) eingegeben wird, der die Amplitude des Magnetfeldes (30) und/oder die Frequenz und/oder die Wiederholdauer und/oder die Behandlungszeit und/oder die Impulsform steuert.

2. Vorrichtung nach Anspruch 1, **dadurch gekennzeichnet, dass** während der Längsverschiebung die Verweildauer an einer bestimmten Position der mindestens einen längsverschiebbaren Magnetspule (8, 8a, 8b) positionsabhängig in Abhängigkeit von der Region des Körpers der Person (3) steuerbar ist.

3. Vorrichtung nach Anspruch 1 oder 2, **dadurch gekennzeichnet, dass** während der Längsverschiebung die Wiederholfrequenz der Einwirkung des Magnetfeldes (30) an einer bestimmten Position der mindestens einen längsverschiebbaren Magnetspule (8, 8a, 8b) positionsabhängig von der Region des Körpers der Person (3) steuerbar ist.

4. Vorrichtung nach einem der Ansprüche 1 bis 3, **dadurch gekennzeichnet, dass** die Längsverschiebung der Magnetspule (8) mit einer drehend angetriebenen Spindelstange (13) erfolgt, welche mindestens eine Spindelmutter (15) durchgreift, die mit der mindestens einen Magnetspule (8, 8a, 8b) verbunden ist.

5. Vorrichtung nach einem der Ansprüche 1 bis 3, **dadurch gekennzeichnet, dass** die Längsverschiebung der mindestens einen Magnetspule (8, 8a, 8b) mit einem in der Länge veränderbaren Zugseil (39) erfolgt.

6. Vorrichtung nach einem der Ansprüche 1 bis 5, **dadurch gekennzeichnet, dass** die Längsverschiebung der mindestens einen Magnetspule (8, 8a, 8b) mit einem Antriebsmotor (43) erfolgt, der in einer die Magnetspule (8, 8a, 8b) aufnehmenden Kassette (7, 7a, 7b) angeordnet ist und der beidseitig angeordnete Ritzel (45) antreibt, welche sich auf mit Zahnstangen (46) versehenen Führungsschienen (47) an der Behandlungsliege (2, 12) abwälzen.

7. Vorrichtung nach einem der Ansprüche 1 bis 6, **dadurch gekennzeichnet, dass** die Behandlungsliege (2, 12) und/oder der Behandlungsstuhl (42) aus mindestens einem schwenkbaren Kopfteil (17) besteht, das mit einem Mittelteil (18) schwenkbar verbunden ist und dass am Mittelteil (18) ein Fußteil (19) schwenkbar ansetzt.

8. Vorrichtung nach einem der Ansprüche 1 bis 7, **dadurch gekennzeichnet, dass** die eine oder die mehreren Magnetspulen (8, 8a, 8b) zusätzlich auch quer zur Längsachse der Behandlungsliege oder des Behandlungsstuhls verschiebbar sind.

9. Vorrichtung nach einem der Ansprüche 1 bis 8, **dadurch gekennzeichnet, dass** die Leistung muskulärer Körperarbeit an der Behandlungsliege (2, 12) und/oder dem Behandlungsstuhl (42) ergometrische Übungsgeräte angeordnet sind.

10. Vorrichtung nach Anspruch 9, **dadurch gekennzeichnet, dass** die ergometrischen Übungsgeräte aus einem oder mehreren Gummiseilen und/oder federbelasteten, hand- oder fußbetätigbaren Schwenkhebeln und/oder Gewichtsstangen und/oder einem Fahrrad-Ergometer bestehen.

11. Vorrichtung nach einem der Ansprüche 1 bis 10, **dadurch gekennzeichnet, dass** eine Chipkarte (60) zur Programmierung des Generators (54) vorgesehen ist, auf der sämtliche Behandlungsdaten pro Körperteil der Person (3) eingespeichert sind, und das Programm derart ausgebildet ist, dass eine körperteil-individuelle Einstellung des Magnetfeldes (30) während der automatischen Verschiebung der einen oder mehreren Magnetspulen (8, 8a, 8b) längs der Behandlungsliege und/oder des Behandlungsstuhles erfolgt.

## Claims

1. Device for operating a magnetic field applicator (1, 10) for magnetic field treatment of people (3) on a treatment bed (2, 12) and/or a treatment chair (42) having at least one magnetic coil (8, 8a, 8b), wherein the at least one magnetic coil (8, 8a, 8b) is designed to be longitudinally displaceable at least partly over the length of the treatment bed (2, 12) and/or of the treatment chair (42) and a magnetic field (30) at least partly penetrating the body of the person (3) can be generated, **characterised in that** during the longitudinal displacement of the at least one magnetic coil (8, 8a, 8b), the strength of the at least one magnetic field (30) and/or its frequency is controllable depending on position as a function of the region of the body of the person (3), wherein the displaceable magnetic coil (8, 8a, 8b) is connected to a position sensor (58) which records the current place and position of the magnetic coil (8, 8a, 8b) with respect to the treated body region of the person (3), and the thus recorded sensor signal is input to a generator (54) which controls the amplitude of the magnetic field (30) and/or the frequency and/or the repetition period and/or the treatment time and/or the pulse form.

2. Device according to claim 1, **characterised in that** during the longitudinal displacement, the residence time at a certain position of the at least one longitudinally displaceable magnetic coil (8, 8a, 8b) is controllable depending on position as a function of the region of the body of the person (3).

3. Device according to claim 1 or 2, **characterised in that** during the longitudinal displacement, the repetition frequency of the action of the magnetic field (30) at a certain position of the at least one longitudinally displaceable magnetic coil (8, 8a, 8b) is controllable depending on position of the region of the body of the person (3).

4. Device according to one of claims 1 to 3, **characterised in that** the longitudinal displacement of the magnetic coil (8) is effected using a rotary driven spindle rod (13) which engages through at least one spindle nut (15) which is connected to the at least one magnetic coil (8, 8a, 8b).

5. Device according to one of claims 1 to 3, **characterised in that** the longitudinal displacement of the at least one magnetic coil (8, 8a, 8b) is effected using a traction rope (39) with changeable length.

6. Device according to one of claims 1 to 5, **characterised in that** the longitudinal displacement of the at least one magnetic coil (8, 8a, 8b) is effected using a drive motor (43) which is arranged in a box (7, 7a, 7b) receiving the magnetic coil (8, 8a, 8b) and which drives pinions (45) arranged on both sides and which are rolled on guide rails (47) provided with racks (46) on the treatment bed (2, 12).

7. Device according to one of claims 1 to 6, **characterised in that** the treatment bed (2, 12) and/or the treatment chair (42) consists of at least one pivotable head part (17) which is connected to a central part (18) to be pivotable and **in that** a foot part (19) attaches to the central part (18) to be pivotable.

8. Device according to one of claims 1 to 7, **characterised in that** the one or more magnetic coils (8, 8a, 8b) are additionally also displaceable transversely to the longitudinal axis of the treatment bed or of the treatment chair.

9. Device according to one of claims 1 to 8, **characterised in that** to achieve muscular body work, ergometric exercise equipment is arranged on the treatment bed (2, 12) and/or the treatment chair (42).

10. Device according to claim 9, **characterised in that** the ergometric exercise equipment consists of one or more elastic cables and/or spring-loaded, hand- or foot- operatable pivot levers and/or weight bars and/or a bicycle ergometer.

11. Device according to one of claims 1 to 10, **characterised in that** a chip card (60) for programming the generator (54) is provided, on which all treatment data per body part of the person (3) are stored, and the program is designed such that an adjustment of the magnetic field (30) depending on the body part is effected during automatic displacement of the one or more magnetic coils (8, 8a, 8b) along the treatment bed and/or the treatment chair.

## Revendications

1. Dispositif pour le fonctionnement d'un applicateur de champ magnétique (1, 10) pour le traitement par champ magnétique de personnes (3) sur une table de traitement (2, 12) et/ou une chaise de traitement (42) comprenant au moins une bobine magnétique (8, 8a, 8b), dans lequel la au moins une bobine magnétique (8, 8a, 8b) est formée de manière à pouvoir coulisser au moins partiellement de façon longitudinale sur la longueur de la table de traitement (2, 12) et/ou de la chaise de traitement (42), et un champ magnétique (30) traversant au moins partiellement le corps de la personne (3) peut être produit, **caractérisé en ce que** pendant le coulissement longitudinal de la au moins une bobine magnétique (8, 8a, 8b) l'intensité du au moins un champ magnétique (30) et/ou sa fréquence sont commandables selon la position, en fonction de la région du corps de la personne (3), dans lequel la bobine magnétique coulissante (8, 8a, 8b) est reliée à un capteur de position (58) qui détecte la situation et la position actuelles de la bobine magnétique (8, 8a, 8b) par rapport à la région du corps traité de la personne (3), et le signal de capteur ainsi détecté est entré dans un générateur (54) qui commande l'amplitude du champ magnétique (30) et/ou la fréquence et/ou la durée de répétition et/ou le temps de traitement et/ou la forme d'impulsion.

2. Dispositif selon la revendication 1, **caractérisé en ce que** pendant le coulissement longitudinal, la durée de séjour à une position déterminée de la au moins une bobine magnétique coulissant longitudinalement (8, 8a, 8b) est commandable selon la position en fonction de la région du corps de la personne (3).

3. Dispositif selon la revendication 1 ou 2, **caractérisé en ce que** pendant le coulissement longitudinal, la fréquence de répétition de l'action du champ magnétique (30) à une position déterminée de la au moins une bobine magnétique coulissant longitudinalement (8, 8a, 8b) est commandable selon la position de la région du corps de la personne (3).

4. Dispositif selon l'une des revendications 1 à 3, **caractérisé en ce que** le coulissement longitudinal de la bobine magnétique (8) se fait avec une broche filetée (13) entraînée en rotation, laquelle vient en prise à travers au moins un écrou de broche (15) qui est relié à la au moins une bobine magnétique (8, 8a, 8b).

5. Dispositif selon l'une des revendications 1 à 3, **caractérisé en ce que** le coulissement longitudinal de la au moins une bobine magnétique (8, 8a, 8b) se fait avec un câble de traction (39) à longueur variable.

6. Dispositif selon l'une des revendications 1 à 5, **caractérisé en ce que** le coulissement longitudinal de la au moins une bobine magnétique (8, 8a, 8b) se fait avec un moteur d'entraînement (43) qui est disposé dans un caisson (7, 7a, 7b) recevant la bobine magnétique (8, 8a, 8b) et qui entraîne des pignons (45), disposés des deux côtés, qui roulent sur des rails de guidage (47) pourvus de crémaillères (46) sur la table de traitement (2, 12).

7. Dispositif selon l'une des revendications 1 à 6, **caractérisé en ce que** la table de traitement (2, 12) et/ou le chaise de traitement (42) se composent d'au moins une partie de tête pivotante (17), qui est reliée de manière pivotante à une partie centrale (18), et qu'une partie de pied (19) est rapportée de manière pivotante sur la partie centrale (18).

8. Dispositif selon l'une des revendications 1 à 7, **caractérisé en ce que** la une ou plusieurs bobines magnétiques (8, 8a, 8b) sont en outre aptes à coulisser en supplément transversalement par rapport à l'axe longitudinal de la table de traitement ou de la chaise de traitement.

9. Dispositif selon l'une des revendications 1 à 8, **caractérisé en ce que** pour fournir un travail corporel musculaire, des appareils d'exercice ergométriques sont disposés sur la table de traitement (2, 12) et/ou le chaise de traitement (42)

10. Dispositif selon la revendication 9, **caractérisé en ce que** les appareils d'exercice ergométriques se composent d'un ou plusieurs câbles en caoutchouc et/ou leviers pivotants contraints par ressort, à actionner avec les mains ou les pieds, et/ou de barres lestées et/ou d'un ergomètre de bicyclette.

11. Dispositif selon l'une des revendications 1 à 10, **caractérisé en ce qu'**il est prévu une carte à puce (60) pour la programmation du générateur (54), sur laquelle sont mémorisées toutes les données de traitement pour chaque partie du corps de la personne (3), et le programme est conçu de telle sorte qu'un réglage individuel par partie de corps du champ magnétique (30) se fasse pendant le coulissement automatique de la une ou plusieurs bobines magnétiques (8, 8a, 8b) le long de la table de traitement et/ou du chaise de traitement.
